# EUROPÄISCHE PATENTSCHRIFT

(11) **EP 0 648 117 B1**
(45) Veröffentlichungstag und Bekanntmachung des Hinweises auf die Patenterteilung: **23.07.1997**
(21) Anmeldenummer: 93912628.0
(22) Anmeldetag: 22.06.1993
(51) Int. Cl.: A61K 31/00

(54) **ARZNEIMITTEL ZUR VERHINDERUNG DER TOLERANZENTWICKLUNG WÄHREND DER BEHANDLUNG MIT BENZODIAZEPIN-REZEPTOR-BINDENDEN WIRKSTOFFEN**
DRUGS FOR PREVENTING THE DEVELOPMENT OF TOLERANCE DURING TREATMENT WITH BENZODIAZEPINE-RECEPTOR-BINDING SUBSTANCES
MEDICAMENT VISANT A EMPECHER L'ACCOUTUMANCE LORS DE TRAITEMENTS A L'AIDE DE SUBSTANCES ACTIVES FIXANT LES RECEPTEURS AUX BENZODIAZEPINES

(30) Priorität: 09.07.1992 DE 4222826
(43) Veröffentlichungstag der Anmeldung: 19.04.1995
(73) Patentinhaber: SCHERING AKTIENGESELLSCHAFT, 13353 Berlin (DE)
(72) Erfinder: TURSKI, Lechoslaw, D-1000 Berlin 20 (DE); STEPPUHN, Karin, Gabriele, D-1000 Berlin 41 (DE); SCHNEIDER, Herbert, D-1000 Berlin 15 (DE); STEPHENS, David, Norman, D-1000 Berlin 19 (DE)
(86) Internationale Anmeldenummer: DE9300561
(87) Internationale Veröffentlichungsnummer: WO9401094

(56) Entgegenhaltungen:
- EP-A- 0 488 959
- EP-A- 0 514 023
- EP-A- 0 527 540
- EP-A- 0 530 446
- The Merck Manual, 15th ed., Seiten 1476-1495

## Beschreibung

Die Erfindung betrifft die Verwendung von NMDA-Antagonisten oder deren physiologischen Salzen für die Herstellung von Arzneimitteln zur Verhinderung der Toleranzentwicklung während der Behandlung mit Benzodiazepin-Rezeptor-bindenden Wirkstoffen.

Sowohl in der Klinik als auch in der Praxis werden bei Krampfleiden und Schlafstörungen bzw. zur Sedierung und Angstlösung häufig chronische Behandlungen mit benzodiazepinrezeptorbindenden Pharmaka, wie z.B. Diazepam (Valium), durchgeführt. Das größte Problem für die Patienten sind die während der Therapie auftretende Toleranz und die nach Absetzen dieser Stoffe auftretenden Entzugserscheinungen wie Muskelsteifigkeit, Tremor, Krämpfe und Angstzustände, unter denen die Patienten leiden.

Die Rolle von exzitatorischen Aminosäuren im Zentralnervensystem hat in den letzten Jahren zunehmendes Interesse gewonnen. So wurde Glutamat als Neurotransmitter identifiziert und drei weitere Rezeptorsubtypen für exzitatorische Aminosäuren gefunden und charakterisiert, die nach den spezifisch wirksamen, Glutamat-analogen Aminosäuren N-Methyl-D-Aspartat (NMDA), Kainat- und Quisqualat-Rezeptoren benannt wurden.

EP-A-488959 offenbart die Verwendung von NMDA-Antagonisten zur Behandlung von Entzugserscheinungen beim Absetzen von u.a. Chlordiazepoxid und Diazepam.

Überraschenderweise wurde gefunden, daß excitatorische Aminosäuren an der Entstehung der Toleranz gegenüber Benzodiazepin-Rezeptor-bindenden Wirkstoffen beteiligt sind und daß die Blockade des NMDARezeptors die Entwicklung der Toleranz verhindert oder reduziert.

Gegenstand der Erfindung ist die Verwendung von NMDA-Antagonisten oder ihren physiologisch verträglichen Salzen für die Herstellung von Arzneimitteln zur Verhinderung der Toleranzentwicklung, die während der Therapie mit Benzodiazepin-Rezeptor-bindenden Wirkstoffen entsteht.

Die Methoden zur Feststellung der Toleranz basieren auf der Messung der explorativen lokomotorischen Aktivität bei Mäusen während chronischer Behandlung mit BDZ-Rezeptor-bindenden Wirkstoffen wie am Beispiel von Benzodiazepinen gezeigt wird.

Die Unterdrückung der explorativen Aktivität gehört zu den bekanntesten sedierenden Wirkungen. Während einer Langzeittherapie mit BDZ tritt an Mäusen ein Verlust der sedierenden Wirkung ein. Um die Toleranzentwicklung während der chronischen Gabe von Benzodiazepinen zu untersuchen, wurden männliche NMRI Mäuse mit einem Gewicht von 20 - 24 g unter täglichen kontrollierten Verhältnissen (6.00 - 18.00 Uhr Hell/Dunkelrythmus, 45-55 % Luftfeuchte und freiem Zugang zu Wasser und Futter) 12 Tage lang mit 15 mg/kg Diazepam in Sesamöl behandelt. Die Kontrolltiere wurden unter gleichen Bedingungen 12 Tage lang mit dem Vehikel s.c. behandelt. Die zwölftägige Behandlung mit 15 mg/kg Diazepam führt bei Mäusen zu einer totalen Toleranz in der sedierenden Eigenschaft.

Um die Rolle der excitatorischen Aminosäuren in der Toleranzentwicklung zu untersuchen, wurden die Tiere mit NMDA-Antagonisten behandelt. Hierbei wurden unter einer leichten Äthernarkose osmotisch wirkende Minipumpen intraperitoneal implantiert und das Verhalten von Mäusen in der Lokomotionsanlage von Tag eins bis Tag zwölf untersucht. Die Pumpen wurden vorher mit NMDA-Antagonisten gefüllt. Die Pumpenrate betrug über 12 Tage 1 mg/kg/h.

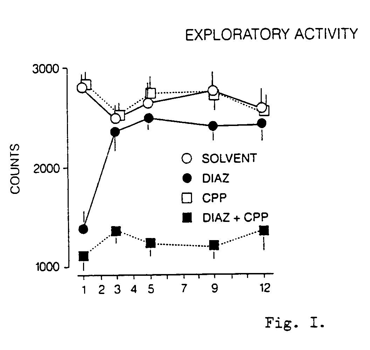

Durch die Behandlung mit NMDA-Antagonisten während der Gabe von Diazepam wird die Entstehung der Toleranz verhindert wie am Beispiel mit CPP gezeigt wird (Fig. 1).

Diesen Untersuchungen ist zu entnehmen, daß die Blockade des NMDA-Rezeptors während der Behandlung mit BDZ-bindenden Wirkstoffen ausreichend ist, um die Entwicklung der Toleranz auf die sedierende Wirkung zu verhindern.

Erfindungsgemäß geeignet sind:

**Die kompetitiven NMDA-Antagonisten** -2-Amino-7-phosphonoheptansäure (AP 7) und Analoga; 3-((+₋)2-Carboxy-piperazin-4-yl)-propyl-1-phosphonsäure (CPP) und Analoga; (e)-4-(3-Phosphonoprop-2-enyl)piperazin-2-carbonsäure (CPPene) und Analoga; cis-4-Phosphonomethyl-2-piperidincarbonsäure (CGS 19755); DL-(E)-2-Amino-4-methyl-5-phosphono-3-pentansäure, (CGP 40116); Enantiomere und Analoga;
S-α-Amino-5-phosphonomethyl-[1,1'-biphenyl]-3-propansäure,
E-2-Amino-4-methyl-5-phosphono-3-pentensäure,
E-2-Amino-4-methyl-5-phosphono-3-pentensäure-ethylester,
cis-4-Phoshonomethyl-2-piperidincarbonsäure,
(R)-4-Oxo-2-amino-5-phosphono-pentansäure,
2-Amino-4,5-(1,2-cyclohexyl)-7-phosphonoheptansäure,
4-(Phosphonomethyl)-DL-phenylglycin,
4-(3-Phosphonopropyl)-2-piperidincarbonsäure,
2-(2-Phosphonoethyl)-DL-phenylalanin,
3-Carboxy-5-(phosphonoethyl)-1,2,3,4-tetrahydroisoquinolin,
3-Carboxy-5-phosphono-1,2,3,4-tetrahydroisoquinolin,
cis-DL-4-[(1(2)H-Tetrazol-5-yl)methyl]2-piperidincarbonsäure,
cis-4-(3-Phosphonoprop-1-enyl)-2-piperidincarbonsäure,
E-2-Amino-4-propyl-5-phosphono-3-pentensäure,
Phosphorsäure-4-(2-carboxy-piperidinyl)ester und
1-[4(4-chloro-α,α-dimethylbenzyl)-2-methoxyphenyl]-1,2,4-triazol-3-carbonsäureamid;

**Nicht-kompetitive NMDA-Antagonisten** (+)10,11-Dihydro-5-methyl-5H-dibenzo-[a,d]cycloheptan-5,10imin (MK-801) und Analoga; Memantine und andere Amantadin-Analoga; Ketamin und Analoga, Budipin und Analoga; Ifenprodil und Analoga; **Antagonisten der Glycinbindungsstelle** -Kynurensäure und Analoga; 1-Hydroxy-3-aminopyrrolidin-2-on (HA-966) und Analoga;**Polyamine Spermin und Spermidin und Analoga;**Hemmer der excitatorischen Aminosäuren-Synthese.

Kompetitive NMDA-Antagonisten sind als bevorzugt zu betrachten.

Als Benzodiazepin-Rezeptor-bindende Wirkstoffe seien Benzodiazepine und β-Carboline genannt wie beispielsweise Diazepam, Nitrazepam, Triazolam, Chordiazepoxid.

Die vorliegende Erfindung umfaßt auch die Kombination von NMDA-Antagonisten oder deren Salze mit Benzodiazepin-bindenden Wirkstoffen zur Verhinderung der Toleranzentwicklung bei der Therapie mit sedierenden Stoffen.

Die Erfindung umfaßt auch die Verwendung pharmazeutischer Mittel, die die genannten Verbindungen in einer wirksamen Menge enthalten, zur Herstellung von Arzneimitteln zur Prophylaxe der Toleranzentwicklung auf BDZ-Rezeptor-bindende Wirkstoffe. Die Arzneimittel werden nach an sich bekannten Verfahren hergestellt, indem man den Wirkstoff mit geeigneten Träger Hilfs-und/oder Zusatzstoffen in die Form eines pharmazeutischen Präparates bringt, das insbesondere für die enterale oder parenterale Applikation geeignet ist.

Die Applikation kann oral oder sublingual als Feststoff in Form von Kapseln oder Tabletten oder als Flüssigkeit in Form von Lösungen, Suspensionen, Elixieren oder Emulsionen oder rektal in Form von Suppositorien oder in Form von gegebenenfalls auch subcutan anwendbaren Injektionslösungen erfolgen. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen oder als Depotzubereitung formuliert sein.

Als Trägersysteme können auch grenzflächennahe Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,001 - 0,034 mg, wobei die Dosis als einmal zu verabreichende Einzeldosis oder unterteilt in 2 oder mehrere Tagesdosen gegeben werden kann.

In den erfindungsgemäßen Kombinationspräparaten können die Wirkstoffe in einer Formulierung oder auch in jeweils getrennten Formulierungen vorliegen, wobei die gesamte Dosis einmalig verabreicht oder in mehrere Dosen geteilt wird.

## Patentansprüche

1. Verwendung von kompetitiven und nicht kompetitiven NMDA-Antagonisten oder deren physiologisch verträgliche Salze für die Herstellung von Arzneimitteln zur Prophylaxe der Toleranzentwicklung auf Benzodiazepin-Rezeptor-bindende Wirkstoffe.

2. Verwendung nach Anspruch 1, dadurch gekennzeichnet, daß die Behandlung zusammen mit Benzodiazepin-Rezeptor-bindenden Wirstoffen einsetzt.

3. Verwendung nach Anspruch 1-2, dadurch gekennzeichnet, daß der nicht kompetitive NMDA-Antagonist ein Antagonist der Glycinbindungstelle ist.

4. Verwendung nach Anspruch 1-3, dadurch gekennzeichnet, daß der NMDA-Antagonist ein kompetitiver NMDA-Antagonist ist.

## Claims

1. Use of competitive and non-competitive NMDA antagonists or the physiologically tolerable salts thereof for the manufacture of medicaments for the prophylaxis of the development of tolerance for benzodiazepine-receptor-binding active ingredients.

2. Use according to claim 1, characterised in that the treatment is used together with benzodiazepine receptor-binding active ingredients.

3. Use according to claim 1 or claim 2, characterised in that the non-competitive NMDA antagonist is an antagonist of the glycine-binding site.

4. Use according to claim 1 or claim 2, characterised in that the NMDA antagonist is a competitive NMDA antagonist.

## Revendications

1. Utilisation d'antagonistes du NMDA compétitifs et non compétitifs ou de leurs sels physiologiquement tolérés, pour préparer des médicaments destinés à la prophylaxie du développement de la tolérance à des principes actifs fixant le récepteur des benzodiazépines.

2. Utilisation selon la revendication 1, caractérisée en ce que le traitement utilise en même temps des principes actifs fixant le récepteur des benzodiazépines.

3. Utilisation selon la revendication 1 à 2, caractérisée en ce que l'antagoniste du NMDA non compétitif est un antagoniste du site de liaison de la glycine.

4. Utilisation selon la revendication 1 à 3, caractérisée en ce que l'antagoniste du NMDA est un antagoniste du NMDA compétitif.
